# EUROPÄISCHE PATENTSCHRIFT

(11) **EP 3 236 476 B1**
(45) Veröffentlichungstag und Bekanntmachung des Hinweises auf die Patenterteilung: **04.11.2020**
(21) Anmeldenummer: 17165912.1
(22) Anmeldetag: 11.04.2017
(51) Int. Cl.: G21F 3/00, A61B 6/10

(54) **STRAHLENSCHUTZVORHANG**
RADIATION PROTECTION CURTAIN
RIDEAU DE PROTECTION CONTRE LE RAYONNEMENT

(30) Priorität: 18.04.2016 DE 102016107126
(43) Veröffentlichungstag der Anmeldung: 25.10.2017
(73) Patentinhaber: WIPOTEC GmbH, 67657 Kaiserslautern (DE)
(72) Erfinder: Thomas, Werner, 67691 Hochspeyer (DE); Allmann, Manuel, 67693 Fischbach (DE)
(74) Vertreter: Eder Schieschke & Partner mbB

(56) Entgegenhaltungen:
- JP-A- 2002 228 601
- JP-A- 2015 059 813
- US-A1- 2015 262 720

## Beschreibung

Die vorliegende Erfindung betrifft einen Strahlenschutzvorhang, insbesondere zur Abschirmung von Röntgenstrahlung oder Gammastrahlung. Derartige Strahlenschutzvorhänge dienen üblicherweise der Abdeckung eines Zugangs oder Ausgangs eines Raumes in welchem Objekte mittels Strahlung untersucht werden. In jüngerer Vergangenheit werden neben oben genannten Strahlungsarten auch Terahertz-Strahlen bzw. Terahertz-Wellen zur Untersuchung, beispielsweise in Form von Körperscanner, angewendet.

In der Praxis wird insbesondere Röntgenstrahlung in vielen Bereichen immer häufiger eingesetzt, beispielweise im Bereich der Sicherheit an Flughäfen zur Überprüfung des Inhalts von Koffern. In der Medizin wird die Röntgenstrahlung zudem in einigen Therapien verwendet. Die Röntgenstrahlen durchleuchten den Körper und visualisieren bestimmte Erkrankungen, zum Beispiel Knochenbrüche. Ebenso wird die Röntgenstrahlung in der industriellen Inspektionstechnik eingesetzt. Aufgrund der gesundheitlichen Gefahren für Personen, die die Röntgenanlagen bedienen, ist es erforderlich sich gegen unzulässige Röntgenstrahlen zu schützen.

Eine in der Industrie verbreitete Lösung hierfür ist die Benutzung von Strahlenschutzvorhängen, die vorteilhafterweise eine Bewegung eines zu untersuchenden Objekts, beispielsweise mittels eines Förderbandes, durch einen Strahlenschutzvorhang hindurch ermöglichen. Insbesondere kann hierbei eine Inspektion mehrerer aufeinanderfolgender Objekte (insbesondere verpackter Produkte) erfolgen, wobei die Objekte nacheinander durch den Strahlenschutzvorhang (Eingang) hindurch in einen strahlengeschützten Untersuchungsraum hinein transportiert und vorzugsweise kontinuierlich durch einen weiteren Strahlenschutzvorhang (Ausgang) aus diesem Untersuchungsraum wieder hinausgefördert werden.

Aus der EP 2 194 373 A1 ist beispielsweise ein Strahlenschutzvorhang bekannt, dessen Abschirmungselemente eine gesamte Einheit bilden. Diese Elemente sind auf eine horizontale Stange aufgeschoben. Nachteiligerweise kann ein derartiger Strahlenschutzvorhang durch insbesondere häufigen mechanischen Kontakt mit hindurchtransportierten Objekten beschädigt werden. Tritt eine Beschädigung auch nur eines einzelnen Elements auf, muss die gesamte Einheit ausgewechselt werden. Weiterhin sorgen die Kontaktflächen der Halterungen auf der Stange für eine hohe Reibungskraft, die einem Objekt bei einem Transport durch den Schutzvorhang hindurch und das hierdurch bewirkte Ausschwenken des Schutzvorhangs in Transportrichtung sowie nach oben und/oder zur Seite als Kraft entgegenwirkt.

Aus der EP 2 930 719 A1 ist weiterhin ein Strahlenschutzvorhang bekannt, der eine Vielzahl von geraden vertikalen länglichen benachbarten Abschirmungselementen aufweist. Diese sind auf eine horizontale Stange aufgeschoben. Tritt eine Beschädigung eines Elements auf, müssen zum Auswechseln eines Elements viele andere Elemente entfernt werden. Aus der JP 2002 228601 A ist zudem ein Strahlenschutzvorhang gemäß dem Oberbegriff des Anspruchs 1 bekannt.

Mechanische Beschädigungen eines Strahlenschutzvorhangs, insbesondere der Verschleiß der Oberfläche, und damit das Risiko eines Strahlungslecks sowie das Auftreten eines unerwünschten oder sogar giftigen Abriebs (beispielsweise Bleimehl) und das Risiko der Kontaminierung, insbesondere von Lebensmitteln, sind aber bei der Untersuchung einer Vielzahl von (beispielsweise durch ein Förderband geförderten) Objekten eines Produktstroms nicht auszuschließen. Beispielweise können zu untersuchende Objekte oder Produkte scharfe Kanten aufweisen, die in einer industriellen Massenproduktion mehrfach pro Minute an derselben Stelle der Abschirmungselemente anstoßen. Ein auftretender Schaden muss aber zwingend behoben werden, um den Austritt von Strahlung zu verhindern.

Der vorliegenden Erfindung liegt daher die Aufgabe zugrunde, vorstehend erläuterte Nachteile zu vermeiden und einen Strahlenschutzvorhang zu schaffen, der ein schnelles Verschleißen vermeidet.

Diese Aufgabe wird erfindungsgemäß durch einen Strahlenschutzvorhang mit den Merkmalen des Anspruchs 1 gelöst.

Durch eine parallel zu der eigentlichen strahlenschützenden Lage angebrachte und senkrecht zur Transportrichtung angeordnete und in Transportrichtung gesehen unmittelbar vorgelagerte, mechanisch schützende Lage, wird eine Beschädigung (Abrieb, Loch, Riss, etc.) der meist kostenintensiven strahlenschützenden Lage vermindert oder gar verhindert.

Die funktionale Trennung von Strahlenschutz und mechanischem Schutz (gegen Beschädigungen wie Risse, Löcher, Abrieb, etc.) ermöglicht vorteilhafterweise auch eine optimale Materialwahl für die jeweilige Lage. So kann die Materialwahl für die strahlenschützende Lage ohne Berücksichtigung einer mechanischen Beschädigung erfolgen. Ebenso kann die Materialwahl für die mechanisch schützende Lage ohne Berücksichtigung einer strahlenschützenden Eigenschaft allein zur Vermeidung von mechanischen Beschädigungen erfolgen. Entsprechend ist es nach der erfindungsgemäßen Lösung möglich, die Materialwahl inklusive deren Beschichtung für die mechanisch schützende Lage, beispielsweise hinsichtlich Härte und Art (Glattheit) der Oberfläche, Reibwert und Rissfestigkeit, optimal zu treffen, die ein abriebfestes Hindurchbewegen von Objekten ermöglicht. Als Materialien für die mechanisch schützende Lage kommen beispielweise Folien, insbesondere aus Metall oder Kunststoff, sowie gewebte Faser oder ein Geflecht in Frage.

Erfindungsgemäß kann eine Beschädigung der strahlenschützenden Lage selbst bei einem, in der industriellen Anwendung auftretenden, häufigen mechanischen Kontakt mit von beispielsweise 200 bis 300 Packungen pro Minute durch den Strahlenschutzvorhang hindurchtransportierten Objekten vermieden werden.

Die in Bezug zur strahlenschützenden Lage separate bzw. getrennte, eigenständige, mechanisch schützende Lage besteht aus wenigstens einem Segment, das kleinere Abmessungen als die strahlenschützende Lage aufweist. Das wenigstens eine mechanisch schützende Segment ist an der strahlenschützenden Lage auswechselbar angeordnet, so dass bei einer wesentlichen Beschädigung (Loch, Riss, etc.) des mechanisch schützenden Segments vor einer weiterführenden Beschädigung der dahinterliegenden strahlenschützenden Lage das mechanisch schützende Segment auf einfache Art und Weise ausgetauscht bzw. durch ein unbeschädigtes Segment ersetzt werden kann. Ein zeitintensives Demontieren oder gar Zerlegen des gesamten Strahlenschutzvorhangs ist vorteilhafterweise nicht nötig.

Da das wenigstens eine mechanisch schützende Segment geringere Abmessungen (Breite und/oder Höhe) als die strahlenschützende Lage besitzt, können ein oder mehrere Segmente vorteilhafterweise beschränkt an Stellen (insbesondere voraussichtliche Kontaktstellen mit Objekten) eingesetzt werden, an denen mechanische Beschädigungen zu befürchten sind. So kann je nach Transport (einspurig oder mehrspurig) und Art der zu untersuchenden Objekte eines Produktstroms der Strahlenschutzvorhang bedarfsweise mit mechanischen vorgelagerten Segmenten ergänzt werden, ohne dass in jedem Fall die gesamte Breite und/oder Höhe der strahlenschützenden Lage abgedeckt werden muss. Hierdurch kann vorteilhafterweise eine dem Transport entgegenwirkende und auf die Lage der Objekte einwirkende Kraft, welche durch das Eigengewicht und durch das für einen Transport in den strahlengeschützten Raum hinein erforderliche Verschwenken des Strahlenschutzvorhangs entsteht, auf das Notwendigste begrenzt werden.

Für eine Anordnung eines oder mehrerer Segmente an gewünschten Stellen an der strahlenschützenden Lage sind beispielsweise oben liegende Freistellen an einer gemeinsamen Aufhängung oder Lagerung an einer Stange (quer zur Transportrichtung angeordnet) vorgesehen. In diese vorgesehenen Freistellen können Segmente bei Bedarf eingehängt werden.

Selbstverständlich ist es aber auch denkbar, die Segmente direkt an der Oberfläche der strahlenschützenden Lage lösbar anzuordnen, beispielsweise einzuhängen, einzuklippsen etc., wobei an der Oberfläche der strahlenschützenden Lage zu den entsprechenden an den Segmenten vorgesehen Halteelementen komplementäre Halteelemente - wie beispielsweise Ösen, Schlaufen, Ringe, Klettverbinder - vorgesehen sein können. Hierbei ist es auch denkbar, mechanisch schützende Segmente nicht nur in ihrer seitlichen Position (quer zur Transportrichtung), sondern auch in ihrer Höhe in unmittelbarer Nähe oder vorzugsweise direkt (mit flächenförmigem Kontakt) an der strahlenschützenden Lage in unterschiedlichen gewünschten Positionen anzuordnen.

In bevorzugter Ausgestaltung der Erfindung sind die mechanisch schützenden Segmente in unterschiedlichen Abmessungen (Breite und/oder Höhe und/oder Dicke) in form eines Baukastensystems (modulare Segmente) vorhanden, so dass auf alle erdenklichen Objekte sowie deren Lage optimiert werden kann. Hierdurch wird vorteilhafterweise ein bestmöglicher Schutz der strahlenschützenden Lage gegen Beschädigungen gewährleistet, ohne den Transport der Objekte oder deren Lage zu behindern.

In vorteilhafter Ausgestaltung der Erfindung ist der Strahlenschutzvorhang als Schutzvorhang gegen Röntgen- oder Gammastrahlung ausgebildet, da gerade diese Strahlungsarten für Personen (und Tiere) als besonders gesundheitsgefährdend angesehen werden. Selbstverständlich kann der erfindungsgemäße Strahlenschutzvorhang auch bei anderen Strahlungsarten oder Wellen, eingesetzt werden, bei denen ein Austritt von Strahlung, sei es auch nur um deren Wirkung zu begrenzen, unerwünscht ist. So können auch andere Strahlungsarten (Terahertz-, Gigahertz-, UV-Strahlung, etc.), beispielsweise eine Terahertz-Strahlung bei sogenannten Körperscannern, in ihrer Reichweite und Auswirkung mit einem erfindungsgemäßen Strahlenschutzvorhang auf eine vorgesehene Räumlichkeit begrenzt werden.

Neben dem vorstehend erläuterten, segmentartigen, modularen Aufbau der mechanisch schützenden Lage mit der Baukasten-Option von Segmenten in unterschiedlichen Abmessungen, kann auch die strahlenschützende Lage aus mehreren, vorzugsweise seitlich benachbarten Segmenten bestehen bzw. baukastenförmig zusammengestellt und montiert werden. Hierdurch wird vorteilhafterweise eine Größenanpassung der strahlenschützenden Lage, insbesondere in ihren seitlichen Abmessungen und damit quer zur Transportrichtung von Objekten durch den Strahlenschutzvorhang, an die Gegebenheiten (beispielsweise Breite des Förderbandes) ermöglicht.

In besonderer Ausgestaltung der Erfindung können ein oder mehrere Segmente der strahlenschützenden Lage an ihrer oberen Kante ein erstes unteres Scharnierteil, beispielsweise in Form von Ösen, Hülsen oder Schlaufen aufweisen, durch welches ein hierzu komplementäres oberes Scharnierteil (Bolzen, Stift, Draht, Stange, etc.) hindurchgreift. Sind die vorgenannten ersten unteren Scharnierteile im Querschnitt geschlossen (ringförmig) ausgebildet, werden für eine bestimmte Anwendung, vor einem Montieren der gesamten strahlenschützenden Lage, entsprechende Segmente ausgewählt, zusammengestellt und insgesamt montiert.

Sind die ersten unteren Scharnierteile dagegen im Querschnitt nicht geschlossen, sondern (beispielsweise nach unten gerichtet) unterbrochen ausgebildet, können die Segmente auch nachträglich bedarfsweise für eine bestimmte Anwendung oder zu Wartungszwecken geändert bzw. ausgewechselt werden. Hierzu werden in einem bereits montierten bzw. aufgehängten Schutzvorhang einzelne Segmente entfernt (beispielsweise nach oben ausgehängt) und nach einem etwaigen Verschieben ein anderes Segment oder andere Segmente eingehängt.

In weiterer Ausgestaltung der Erfindung weist das wenigstens eine Segment der mechanisch schützenden Lage ein zweites unteres Scharnierteil auf, das mit dem oberen Scharnierteil derart zusammenwirkt, dass auch die mechanisch schützende Lage bzw. dessen wenigstens ein Segment wie die strahlenschützende Lage in Transportrichtung schwenkbar gelagert ist.

In besonderer Ausgestaltung der Erfindung ist die wenigstens eine mechanisch schützende Lage in vorgesehenen Freistellen des ersten unteren Scharnierteils einer bereits installierten strahlenschützenden Lage einhängbar ausgebildet. Hierzu kann das zweite, untere Scharnierteil der mechanisch schützenden Lage beispielsweise ein von oben übergreifendes und nach unten geöffnetes Profil aufweisen.

Um den Transport von zu untersuchenden Objekten durch den Strahlenschutzvorhang zu erleichtern, können die Segmente der strahlenschützenden Lage aus mehreren seitlich benachbarten, separaten Lamellen bestehen, die einzeln verschwenkbar gelagert sind.

Die wenigstens eine strahlenschützende Lage und/oder die wenigstens eine mechanisch schützenden Lage sind vorzugsweise flexibel, insbesondere biegeschlaff ausgebildet. Hier bedeutet biegeschlaff, dass in der mechanisch schützenden Lage keine eigene Gelenkstelle vorhanden ist. Lediglich die Aufhängung kann ein oder mehrere Gelenke aufweisen). Zudem sind die strahlenschützende Lage und die mechanisch schützende Lage vorzugsweise zueinander separat beweglich ausgebildet.

Weitere vorteilhafte Ausgestaltungen ergeben sich aus den abhängigen Ansprüchen.

Die Erfindung wird nachfolgend anhand eines in der Zeichnung dargestellten Ausführungsbeispiels näher erläutert.

In der Zeichnung zeigen:
- Fig. 1: eine perspektivische Darstellung eines erfindungsgemäßen Strahlenschutzvorhangs ohne mechanisch schützende Lage;
- Fig. 2: eine vergrößerte Detailansicht des Ausschnitts D in Fig. 1;
- Fig. 3: eine Vorderansicht auf einen Strahlenschutzvorhang nach Fig. 1 mit vier eingehängten Segmenten einer mechanisch schützenden Lage;
- Fig. 4: eine Vorderansicht auf einen Strahlenschutzvorhang nach Fig. 1 mit zwei äußeren eingehängten Segmenten einer mechanisch schützenden Lage;
- Fig. 5: eine perspektivische Ansicht des Strahlenschutzvorhangs nach Fig. 4;
- Fig. 6: eine vergrößerte Detailansicht des Ausschnitts E in Fig. 5.

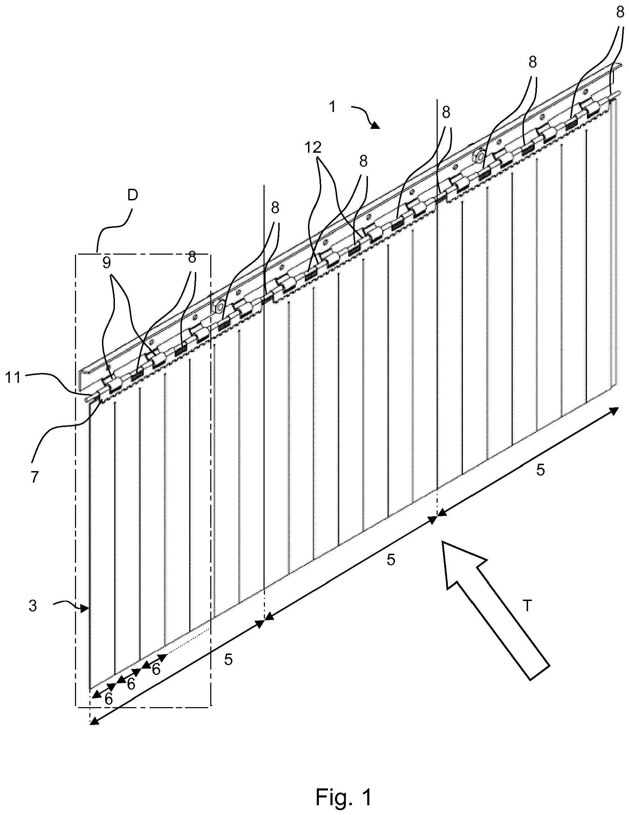

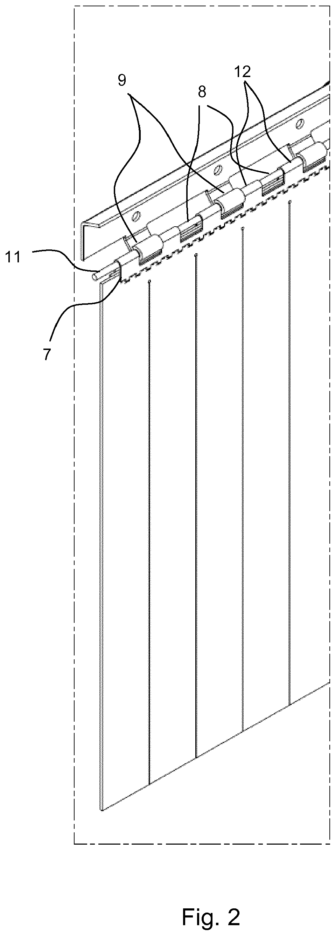

Wie in Fig. 1 und Fig. 2 dargestellt, besteht der erfindungsgemäße Strahlenschutzvorhang 1 aus wenigstens einem (im Beispiel aus drei Segmenten) Segment 5 einer modular aufgebauten strahlenschützenden Lage 3. Die rechteckförmig ausgebildeten Segmente 5 sind seitlich benachbart, quer zu einer Transportrichtung T von zu untersuchenden nicht dargestellten Objekten durch den Schutzvorhang hindurch an ihrer oberen Kante über ein erstes unteres Scharnierteil 7 an einer Stange 11 gelagert. Die Stange 11 ist innerhalb eines oberen Scharnierteils 9 gelagert, so dass der Strahlenschutzvorhang 1 quer zur Transportrichtung T (von zu untersuchenden Objekten eines Produktstroms) über einem in der Zeichnung nicht dargestellten Förderband befestigt werden kann und den Eingang und/oder Ausgang (in Höhe und Breite) eines ebenfalls in der Zeichnung nicht dargestellten strahlengeschützten Raumes strahlenschützend abdeckt.

Durch die dargestellte scharnierartige Lagerung an der Oberkante eines Segments 5 können die Segmente 5 um die Stange 11 verschwenkt werden, so dass durch das Verschwenken in Richtung der Transportrichtung T sowie nach oben (und/oder zur Seite) Objekte durch den Schutzvorhang hindurch gelangen können. Nach dem Hindurchtreten der Objekte schwenkt das wenigstens eine Segment 5 zurück in die in Fig. 1 dargestellte Ausgangslage, in welcher seitlich benachbarte Segmente 5 aneinander anliegen und ein Austreten von unerwünschter Strahlung, insbesondere Röntgenstrahlung, verhindern.

Jedes Segment 5 der strahlenschützenden Lage 3 besteht aus mehreren (beispielsweise sieben) Lamellen 6. Das Material des Segments 5 kann zudem selbst statt formsteif auch flexibel, insbesondere biegeschlaff ausgebildet sein, so dass auch einzelne seitlich benachbarte Lamellen 6 eines Segments 5 in Transportrichtung unabhängig voneinander ausgelenkt werden können.

An seiner oberen Kante ist das Segment 5 in dem ersten unteren Scharnierteil 7 befestigt, beispielsweise mit entsprechenden Werkzeugen eingepresst, und weist mehrere (im Beispiel acht) Haltebereiche in Form von Klammern 12 auf, die die Stange 11 übergreifen. Entsprechend ist es möglich, ein Segment 5 in eine in dem oberen Scharnierteil 9 eingeführte Stange 11 von oben (oder schräg oben) einzuhängen. Wird die Stange 11 dagegen von den Haltebereichen 12 im Querschnitt umschlossen, muss die Stange 11 nachträglich in die Scharnierteile 9 und 7 eingeführt werden.

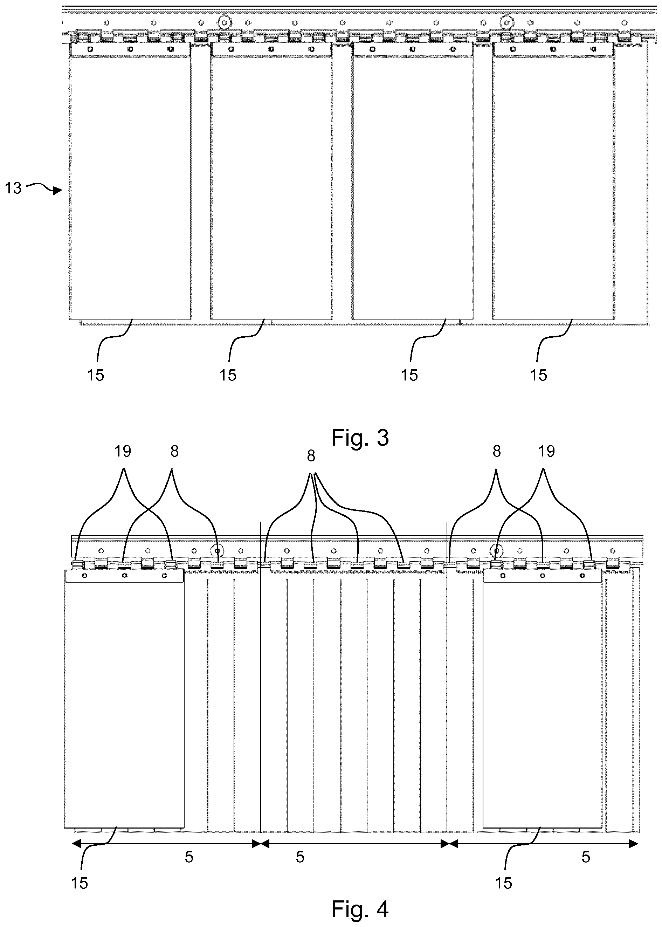

Wie aus Fig. 1 und Fig. 2 ersichtlich, befinden sich an der oberen Kante in dem ersten unteren Scharnierteil 7 zusätzliche (im Beispiel vier) Freistellen 8, die nicht von den Haltebereichen des oberen Scharnierteils 9 belegt sind. Diese Freistellen 8 (beispielsweise jeweils zwischen den Lagerstellen des oberen Scharnierteils 9 angeordnet) dienen der erfindungsgemäßen Anordnung einer separaten, zu der strahlenschützenden Lage 3 koplanaren mechanisch schützenden Lage 13 (siehe Fig. 3), die vorzugsweise flexibel, insbesondere biegeschlaff ausgebildet sein kann. Die Freistellen 8 können, wie dargestellt, seitlich äquidistant, beispielsweise im Abstand von 20 mm ausgebildet sein, so dass ein Segment 15 (siehe Fig. 3) je nach Bedarf in eine exakte Positionen (jeweils um diesen Abstand versetzt) eingehängt werden kann.

Fig. 3 zeigt einen fertig montierten erfindungsgemäßen Strahlenschutzvorhang mit vier seitlich beabstandeten Segmenten 15 der mechanisch schützenden Lage 13, vor einer in Transportrichtung unmittelbar dahinter liegenden (in Fig. 1 und Fig. 2 erläuterten) strahlenschützenden Lage 3.

Anders als bei der strahlenschützenden Lage 3 ist ein seitlicher Abstand zwischen den Segmenten 15 der mechanisch schützenden Lage 13 möglich und bei Beachtung einer seitlichen Mindestabmessung von Objekten (Abstand kleiner als seitliche Mindestabmessung) ohne Nachteil zulässig, da durch diese Abstände keine Strahlung austreten kann.

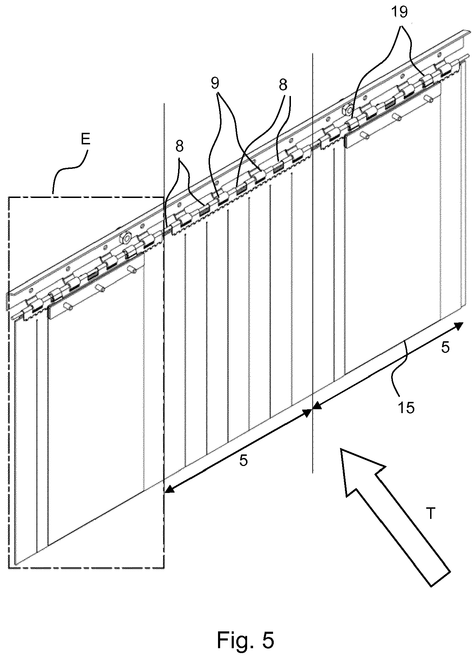

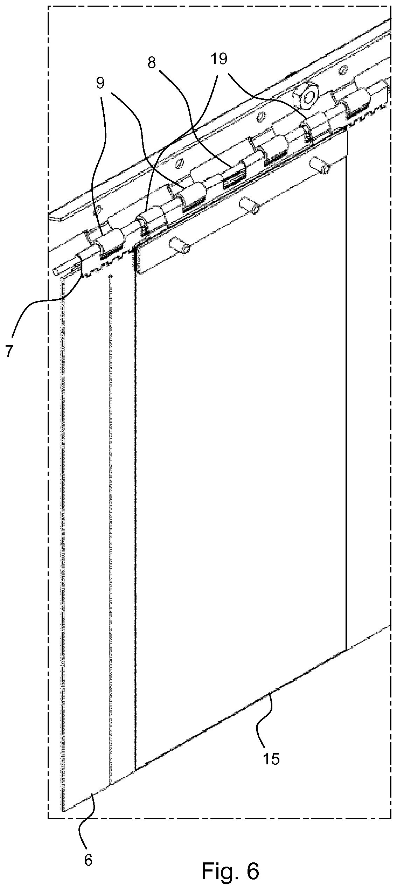

Wie in Fig. 4 dargestellt, können auch nur einzelne beispielsweise äußere Segmente 15 eingehängt sein, beispielsweise bei zweispurig geförderten Objekten entsprechender Abmessung und Spurabstand. Wie insbesondere aus Fig. 5 und 6 ersichtlich, können die Segmente je nach Bedarf um jeweils eine benachbarte Freistelle 8 in eine nach links oder rechts veränderte Lage eingehängt werden, so dass auf den entsprechenden Bedarf (Objektgröße bzw. Spurbreite, Spuranzahl und Spurabstand) mit entsprechend geänderter Position und/oder Anzahl der Segmente 15 reagiert werden kann.

Hierbei müssen die Segmente 15 keineswegs gleichartig ausgebildet sein, sondern können sich insbesondere in ihren seitlichen Abmessungen unterscheiden. Denkbar ist beispielsweise ein Baukastensystem, in dem Segmente 15 unterschiedlicher Breite, beispielsweise in Breiten von 2 cm Unterschied zueinander in mehrfacher Form vorhanden sind.

Die Segmente 5 der strahlenschützenden Lage 3, welche beispielsweise aus mit Bleimehl gefüllten flexiblen einlagigen oder mehrlagigen Lamellen 6 bestehen, können durch die eingehängten Segmente 15 der mechanisch schützenden Lage 13 vor Beschädigungen effektiv geschützt werden.

Wie aus Fig. 3 bis Fig. 6 ersichtlich, können die Segmente 15 der mechanisch schützenden Lage 13 auch derart in ihrer seitlichen Position angeordnet sein, dass sie die Stoßstellen zwischen benachbarten Segmenten 5 der strahlenschützenden Lage 3 überdecken (siehe insbesondere die beiden mittleren Segmente 15 in Fig. 3).

In der bevorzugten Ausgestaltung mit einzelnen, in Transportrichtung T biegsamen, insbesondere biegeschlaffen (beispielsweise 20 mm breiten) Lamellen 6, werden, sobald ein Segment 15 von einem Objekt mit Kraft in Richtung T beaufschlagt wird, die dahinterliegenden Lamellen 6 mit in Richtung T bewegt bzw. verschwenkt.

Die Montage des erfindungsgemäßen Strahlenschutzvorhangs 1 erfolgt bevorzugt durch ein werkzeugloses Einhängen jedes Segments 5 bzw. 15 in das obere Scharnierteil 9 bzw. in die Stange 11 bzw. Freistelle 8. Die Segmente des Strahlenschutzvorhangs sind daher modular einzeln austauschbar.

Weisen die Segmente 5 und/oder 15 an ihren ersten und zweiten unteren Scharnierteilen 7 und 19 nur übergreifende, jedoch nicht die Stange 11 umschließende Haltebereiche auf, so können die Segmente 15 (der mechanisch schützenden Lage 13) und/oder die Segmente 5 (der strahlenschützenden Lage 3) auch an einem bereits fertig montierten Strahlenschutzvorhang 1 nachträglich eingehängt und modular einzeln ausgetauscht werden. Die Segmente 15 der mechanisch schützenden Lage 13 werden an (in dem ersten unteren Scharnierteil 7) vordefinierten Freistellen 8 an der Stange 11 eingehängt, wodurch sich eine definierte Position ergibt. Auf diese Weise sind die Segmente 15 einfach und schnell montierbar.

Durch die im Ausführungsbeispiel dargestellte Art der Montage gibt es keine verlierbaren Teile. Etwaige Schrauben werden an dem oberen Scharnierteil 9 nur zur Montage der Baugruppe selbst bzw. des oberen Scharnierteils 9 an beispielsweise einem strahlengeschützten Raum benötigt.

Durch den erfindungsgemäßen Strahlenschutzvorhang kann vorteilhafterweise ein Schnellwechsel einzelner Segmente 15 (und eventuell auch der Segmente 5) zur Anpassung an beliebige Objekte, insbesondere Packungseigenschaften und Strahlungsstärke (rasche Konfigurierung des optimalen Schutzvorhangs) erfolgen.

### Bezugszeichenliste

- 1: Strahlenschutzvorhang
- 3: strahlenschützende Lage
- 5: Segmente der strahlenschützenden Lage
- 6: Lamellen des Segments 5
- 7: erstes unteres Scharnierteil (der strahlenschützenden Lage)
- 8: Freistelle in dem ersten unteren Scharnierteil 7
- 9: oberes Scharnierteil
- 11: Stange
- 12: Klammer (Haltebereich)
- 13: mechanisch schützende Lage
- 15: Segment der mechanisch schützende Lage
- 19: zweites unteres Scharnierteil (der mechanisch schützenden Lage)
- T: Transportrichtung von zu untersuchenden Objekten
- D: Ausschnitt in Fig. 1
- E: Ausschnitt in Fig. 5

## Patentansprüche

1. Strahlenschutzvorhang, der wenigstens aus zwei zueinander parallel angeordneten Lagen (3, 13) besteht, für einen Transport von zu untersuchenden Objekten eines Produktstroms in einer Richtung (T) durch den Strahlenschutzvorhang hindurch,
wobei
a) wenigstens eine erste Lage als strahlenschützende Lage (3) ausgebildet ist,
b) dieser wenigstens einen ersten strahlenschützenden Lage (3) wenigstens eine hiervon getrennte, eigenständige weitere Lage in Transportrichtung (T) stromaufwärts vorgelagert ist,
c) diese wenigstens eine weitere Lage als mechanisch schützende Lage (13) ausgebildet ist, um die dahinterliegende strahlenschützende Lage (3) vor mechanischen Einwirkungen zu schützen
**dadurch gekennzeichnet, dass**
d) die wenigstens eine weitere mechanisch schützende Lage (13) aus mindestens einem Segment besteht, welches an einer fertig installierten strahlenschützenden Lage (3) an vorgesehenen Stellen einzeln austauschbar angeordnet ist.

2. Strahlenschutzvorhang nach Anspruch 1, **dadurch gekennzeichnet, dass** die mechanisch schützende Lage (13) modular aus mehreren, separaten Segmenten (15) besteht.

3. Strahlenschutzvorhang nach Anspruch 2, **dadurch gekennzeichnet, dass** die mechanisch schützende Lage (13) aus mehreren separaten, in Bezug auf die Transportrichtung (T) quer angeordneten und seitlich benachbarten Segmenten (15) besteht.

4. Strahlenschutzvorhang nach einem der vorhergehenden Ansprüche, **dadurch gekennzeichnet, dass** die wenigstens eine weitere mechanisch schützende Lage (13) geringere Abmessungen als die strahlenschützende Lage (3) besitzt.

5. Strahlenschutzvorhang nach einem der vorhergehenden Ansprüche, **dadurch gekennzeichnet, dass** der Strahlenschutzvorhang als Schutzvorhang gegen Röntgen- oder Gammastrahlung ausgebildet ist.

6. Strahlenschutzvorhang nach einem der vorhergehenden Ansprüche, **dadurch gekennzeichnet, dass** die strahlenschützende Lage (3) aus mehreren separaten, in Bezug auf die Transportrichtung (T) quer angeordneten und seitlich benachbarten Segmenten (5) besteht.

7. Strahlenschutzvorhang nach Anspruch 6, **dadurch gekennzeichnet, dass** einzelne Segmente (5) der strahlenschützenden Lage (3) an einer fertig installierten strahlenschützenden Lage (3) einzeln ohne Demontage der anderen Segmente (5) austauschbar angeordnet sind.

8. Strahlenschutzvorhang nach einem der vorhergehenden Ansprüche, **dadurch gekennzeichnet, dass** die wenigstens eine strahlenschützende Lage (3) mit einem ersten unteren Scharnierteil (7) und/oder die wenigstens eine mechanisch schützende Lage (13) mit einem zweiten unteren Scharnierteil (19) an einem oberen Scharnierteil (9, 11) gelagert ist.

9. Strahlenschutzvorhang nach Anspruch 8, **dadurch gekennzeichnet, dass** die wenigstens eine mechanisch schützende Lage (13) in vorgesehenen vordefinierten Freistellen (8) des ersten unteren Scharnierteils (7) einer bereits installierten strahlenschützenden Lage (3) anordenbar, insbesondere einhängbar ist.

10. Strahlenschutzvorhang nach einem der Ansprüche 1 bis 7, **dadurch gekennzeichnet, dass** die wenigstens eine mechanisch schützende Lage (13) unmittelbar an der strahlenschützenden Lage (3) befestigbar ist.

11. Strahlenschutzvorhang nach einem der Ansprüche 6 bis 10, **dadurch gekennzeichnet, dass** die Segmente (5) der strahlenschützenden Lage (3) aus mehreren seitlich benachbarten Lamellen (6) bestehen.

## Claims

1. Radiation protection curtain, which consists of at least two layers (3, 13) arranged parallel to one another, for transporting objects to be examined of a product stream in a direction (T) through the radiation protection curtain, wherein
a) at least one first layer is designed as a radiation-shielding layer (3),
b) this at least one first radiation-shielding layer (3) is preceded upstream by at least one further independent layer separate from it in the direction of transport (T),
c) this at least one further layer is designed as a mechanically protective layer (13) in order to protect the radiation-shielding layer (3) behind it from mechanical effects,
**characterized in that**
d) the at least one further mechanically protective layer (13) consists of at least one segment, which is arranged on a readily installed radiation-shielding layer (3) at provided points in an individually exchangeable manner.

2. Radiation protection curtain according to claim 1, **characterized in that** the mechanically protective layer (13) consists modularly of several separate segments (15).

3. Radiation protection curtain according to claim 2, **characterized in that** the mechanically protective layer (13) consists of several separate segments (15) arranged transversely with respect to the transport direction (T) and laterally adjacent.

4. Radiation protection curtain according to one of the preceding claims, **characterized in that** the at least one further mechanically protective layer (13) has smaller dimensions than the radiation-shielding layer (3).

5. Radiation protection curtain according to one of the preceding claims, **characterized in that** the radiation protection curtain is designed as a protective curtain against X-rays or gamma radiation.

6. Radiation protection curtain according to one of the preceding claims, **characterized in that** the radiation-shielding layer (3) consists of several separate segments (5) arranged transversely with respect to the direction of transport (T) and laterally adjacent.

7. Radiation protection curtain according to claim 6, **characterized in that** individual segments (5) of the radiation-shielding layer (3) are arranged on a completely installed radiation-shielding layer (3) so as to be exchangeable individually without dismantling the other segments (5).

8. Radiation protection curtain according to one of the preceding claims, **characterized in that** the at least one radiation-shielding layer (3) is mounted on an upper hinge part (9, 11) with a first lower hinge part (7) and/or the at least one mechanically protective layer (13) is mounted on an upper hinge part (9, 11) with a second lower hinge part (19).

9. Radiation protection curtain according to claim 8, **characterized in that** the at least one mechanically protective layer (13) can be arranged, in particular suspended, in provided predefined cut-outs (8) of the first lower hinge part (7) of an already installed radiation-shielding layer (3).

10. Radiation protection curtain according to one of claims 1 to 7, **characterized in that** the at least one mechanically protective layer (13) can be directly attached to the radiation-shielding layer (3).

11. Radiation protection curtain according to one of the claims 6 to 10, **characterized in that** the segments (5) of the radiation-shielding layer (3) consist of several laterally adjacent lamellae (6).

## Revendications

1. Rideau de protection contre les rayons, lequel est constitué d'au moins deux couches (3, 13) disposées parallèlement l'une à l'autre, destiné au transport d'objets d'un flux de produit à examiner dans une direction (T) orientée à travers le rideau de protection contre les rayons, dans lequel
a) au moins une première couche est conçue comme une couche de protection contre les rayons (3),
b) cette au moins une première couche de protection contre les rayons (3) est disposée en amont d'au moins une autre couche séparée et distincte de la première couche, dans la direction de transport (T),
c) cette au moins une autre couche est conçue comme une couche de protection mécanique (13) de façon à protéger la couche de protection contre les rayons (3) située derrière elle contre les effets mécaniques,
**caractérisé en ce que**
d) l'au moins une autre couche de protection mécanique (13) est constituée d'au moins un segment, lequel est discrètement disposé de manière interchangeable sur une couche de protection contre les rayons (3) complètement installée aux emplacements prévus.

2. Rideau de protection contre les rayons selon la revendication 1, **caractérisé en ce que** la couche de protection mécanique (13) est constituée d'une pluralité de segments (15) séparés.

3. Rideau de protection contre les rayons selon la revendication 2, **caractérisé en ce que** la couche de protection mécanique (13) est constituée de plusieurs segments (15) séparés latéralement adjacents, disposés transversalement par rapport à la direction de transport (T).

4. Rideau de protection contre les rayons selon l'une des revendications précédentes, **caractérisé en ce que** l'au moins une autre couche de protection mécanique (13) a des dimensions plus petites que celles de la couche de protection contre les rayons (3).

5. Rideau de protection contre les rayons selon l'une des revendications précédentes, **caractérisé en ce que** le rideau de protection contre les rayons est conçu comme un rideau de protection contre les rayons X ou gamma.

6. Rideau de protection contre les rayons selon l'une des revendications précédentes, **caractérisé en ce que** la couche de protection contre les rayons (3) est constituée de plusieurs segments (5) séparés latéralement adjacents qui sont disposés transversalement par rapport à la direction de transport (T).

7. Rideau de protection contre les rayons selon la revendication 6, **caractérisé en ce que** des segments (5) individuels de la couche de protection contre les rayons (3) sont disposés de manière interchangeable, individuellement et sans démontage des autres segments (5), sur une couche de protection contre les rayons (3) complètement installée.

8. Rideau de protection contre les rayons selon l'une des revendications précédentes, **caractérisé en ce que** l'au moins une couche de protection contre les rayons (3) est montée avec une première partie de charnière (7) inférieure et/ou l'au moins une couche de protection mécanique (13) est montée avec une seconde partie de charnière (19) inférieure sur une partie de charnière (9, 11) supérieure.

9. Rideau de protection contre les rayons selon la revendication 8, **caractérisé en ce que** l'au moins une couche de protection mécanique (13) peut être disposée, notamment suspendue, dans les espaces libres prédéfinis et prévus (8) de la première partie de charnière (7) inférieure d'une couche de protection contre les rayons (3) déjà installée.

10. Rideau de protection contre les rayons selon l'une des revendications 1 à 7, **caractérisé en ce que** l'au moins une couche de protection mécanique (13) peut être fixée directement sur la couche de protection contre les rayons (3).

11. Rideau de protection contre les rayons selon l'une des revendications 6 à 10, **caractérisé en ce que** les segments (5) de la couche de protection contre les rayons (3) sont constitués de plusieurs lamelles latéralement adjacentes (6).
